# EUROPÄISCHE PATENTSCHRIFT

(11) **EP 1 223 182 B1**
(45) Veröffentlichungstag und Bekanntmachung des Hinweises auf die Patenterteilung: **23.04.2003**
(21) Anmeldenummer: 01129911.2
(22) Anmeldetag: 15.12.2001
(51) Int. Cl.: C08F 299/04, C08G 63/08

(54) **Polyester mit (Meth)acrylatendgruppen**
Polyesters with (meth)acrylate end-groups
Polyesters à groupes terminaux méthacrylate

(30) Priorität: 13.01.2001 DE 10101387
(43) Veröffentlichungstag der Anmeldung: 17.07.2002
(73) Patentinhaber: MERCK PATENT GmbH, 64293 Darmstadt (DE)
(72) Erfinder: Siol, Werner, Dr., 64297 Darmstadt (DE); Pokinskyi, Peter, Dr., 64380 Rossdorf (DE)

(56) Entgegenhaltungen:
- EP-A- 0 085 944
- WO-A-98/20839
- DE-A- 2 103 870
- US-A- 4 795 823

## Beschreibung

Die Erfindung betrifft Polyester mit (Meth)acrylatendgruppen gemäß Anspruch 1.

Methacrylsäureester mehrwertiger Alkohole haben als vernetzende Monomerbausteine umfangreiche technische Anwendung gefunden. Zur Abdeckung der unterschiedlichsten technischen Anforderungen wurden in diesem Zusammenhang nahezu alle technisch verfügbaren Alkandiole in die entsprechenden Methacrylsäureester überführt, ebenso ist eine Großzahl der Ethylenglykole und Propylenglykole auch als Methacrylsäureester verfügbar, z. B. Diethylenglykoldimethacrylat oder Polyproylenglykoldimethacrylat.

Demgegenüber spielen Polyesterdiole mit Methacrylatendgruppen oder Acrylatendgruppen (allgemein: (Meth)acrylatendgruppen) nur eine untergeordnete Rolle. Während Polyesterdiole als Weichsegmente für den Aufbau von Polyurethanen von erheblicher Bedeutung sind, haben die entsprechenden Polyesterdiole mit (Meth)acrylatendgruppen keine nennenswerte Bedeutung. Dies ist um so erstaunlicher als diese Polyester in der Regel gut verfügbar sind, gute Weichmacherwirkung, gute optische Eigenschaften und eine gute Witterungsbeständigkeit aufweisen. Dieses Missverständnis ist in erster Linie auf die klassischen Synthesewege für (Meth)acrylsäureester zurückzuführen. So spielt die Synthese von (Meth)acrylsäureester ausgehend von Alkohol und (Meth)acrylsäurechlorid insbesondere wegen der Notwendigkeit, die frei werdende HCI zu binden und aufgrund der Bildung chlorhaltiger Nebenprodukte nur eine untergeordnete Rolle.

In erster Linie erfolgt die Bildung der (Meth)acrylsäureester durch direkte Veresterung der entsprechenden Alkohole mit Methacrylsäure bzw. Acrylsäure oder insbesondere durch Umesterung der Alkohole mit Methylmethacrylat bzw. Methylacrylat mit Hilfe eines basischen oder Organozinnkatalysators (siehe z. B. DD 272067 oder EP 663 386). Bei dieser Veresterung wird naturgemäß auch der Polyester zumindest teilweise umgeestert, so dass eine erhebliche Isomerisierung des Polyesters stattfindet. Demgegenüber reagieren die α, ω- Hydroxygruppen der Polyesterdiole mit Isocyanaten selektiv und schnell, wodurch der Einsatz zum Aufbau von Polyurethanen einfach möglich ist. Auf diesem Wege, so z. B. durch Umsetzung mit Isocyanantoethylmethacrylat oder durch Umsetzung mit einem Diisocyanat und nachfolgender Reaktion mit Hydroxyethylmethacrylat sind Polyesterurethanmethacrylate einfach erhältlich (siehe J. M. S. - Rev. Macromol. Chem. Phys., C33 (2)m 147-180 (1993), Current Status of Urethane (Meth)acrylate Oligomers and Polymers).

Ähnlich selektiv verläuft die Umsetzung von Polyestem mit Carboxylendgruppen mit Glycidylmethacrylat, in diesem Fall erhält man Polyester mit Methacrylatendgruppen, die jedoch Hydroxygruppen enthalten.
Auch die direkte Einführung der Methacryloxylgruppe z. B. im Rahmen einer lebenden Polymerisation zur Synthese von Polymeren mit α, ω-Dimethacrylatgruppen wird beschrieben. So erzeugen Heitz et al. (US-4,412, 063) ein Polyetrahdrofuran mit Methacrylatendgruppen durch kationische Polymerisation in Gegenwart von Methacrylsäureanhydrid.

Kataoka et al. (US-5,929,177) beschreiben den Aufbau eines Blockcopolymeren X-O-(-CH₂-CH₂-O-)ₘ-(Y)ₙ-Z durch lebende Polymerisation ausgehend von einer mehrwertigen, funktionellen, z. B. aminogruppenhaltigen Zentralgruppe X, wobei zunächst ein Polyethylenoxydblock, danach eine Polymethacrylat- oder Polyesterblock Y erzeugt wird und schließlich durch Abbruch mit z. B. Methacrylsäureanhydrid eine Methacryloylgruppe eingeführt wird. Derartige Blockcopolymere mit polymerisierbaren Endgruppen und polarer Zentralfunktion X sind, z. B. zum Aufbau spezieller, polymerisierbarer Tenside geeignet.

Besonders gut biodegradierbare Polyester mit -O-CH₂-O- Einheiten und (Meth)acrylatendgruppen werden von Klaveness et al. beschrieben (US-5,693,321).
Wenn es auf eine spezielle Kettenlängenverteilung und eine hohe Endgruppenfunktionalität nicht ankommt, kann die Einführung der (Meth)acrylatendgruppen auch direkt bei der Synthese der Polyester erfolgen. So beschreibt Vyvoda (US-5,290,852) die Mitverwendung von 2-Hydroxyethyl-methacrylat, Methacrylsäure oder Methacrylsäureanhydrid als monofunktionelle Endgruppen bei der Synthese eines Polyesters auf Basis Glutarsäure. Diese Polyestergemische werden bei der Polymerisation von Vinylchlorid zur Herstellung von innerlich weichgemachtem PVC eingesetzt.
Schmitt et. al. (US-4,795,823) beschreiben die Synthese eines α,ω-Methacrylat-haltigen Polyesters auf Basis Triglykolsäure und T-diol (Kurzbezeichnung für ein komplex aufgebautes Diol) ausgehend von einem Oligoester und Methacrylsäure. Dabei wird der Oligoester mit Methacrylatendgruppen aber auch 32 % des T-diol-dimethacrylates gefunden. Diese Ester-/Polyestermischung wird als Dentalmasse eingesetzt.
Ein erheblicher Anteil kurzkettiger Alkandiolester wird auch der direkten Veresterung von Polyactids mit Methacrylsäure gefunden. Darüber hinaus sind diese durch direkte Veresterung eines Polyactids mit Methacrylsäure erhaltenen Polyester intensiv gefärbt (siehe US-4,731,425 und Vergleichsbeispiel 1).
Eine besonders schonende Synthese von Acrylsäureestern wird von Ayorinde et al. (US-5,491,244) beschrieben. Danach ist ein empfindlicher Epoxyacrylsäureanhydrid zugänglich. Dabei wird ohne jeden Katalysator und mit einem 4-fachen Überschuss an Acrylsäureanhydrid gearbeitet.
Deutlich schlechter verläuft dagegen die Synthese von Methacrylsäureestern ausgehend von Methacrylsäureanhydrid und verschiedenen α-Hydroxycarbonsäureestern (Milchsäureestem). Trotz Katalyse mit Schwefelsäure, einem Überschuss an Methacrylsäureanhydrid und 5h Erhitzen auf 130° C wird nur ein Umsatz von ca. 50 % erreicht. Darüber hinaus bereitet die Abtrennung des nicht umgesetzten Anhydrids Probleme (siehe Rehberg et al., Journal of the American Chemical Society, Vol 67, 210 (1945).

Nach wie vor besteht ein Bedarf an Polyestern mit (Meth)acrylatendgruppen z. B. zum Aufbau von schrumpfarmen Systemen oder im Bereich der Reaktionsklebstoffe. Von besonderem Interesse sind Polyester mit polymerisierbaren (Meth)acrylatendgruppen auf der Basis von Poly-α-hydroxycarbonsäuren (Zum Aufbau von Copolymeren mit abbaubaren Lactid- Ästen siehe z. B. Sandner et al., Macromol. Symp. 1996, 103 (Polymer and Medicine), 149-62).
Zur Gewährleistung einer guten biologischen Abbaubarkeit soll der Gehalt an kurzkettigen Di(meth)acrylaten ohne Estergruppe zwischen den (Meth)acrylatendgruppen möglichst gering sein.
Ebenso besteht Bedarf an möglichst hoch funktionalisierten, chlorfreien, transparenten, optisch hochwertigen amorphen Polyestern mit (Meth)acrylatendgruppen, die ebenso wie die Polyesterdiole der Polyurethane als witterungsstabiles Weichsegment zum Aufbau von Poly(meth)acrylatsystemen geeignet sind.

Ein Bedarf an derart funktionalisierten (Meth)acrylaten besteht für folgende spezielle medizinisch-technische Anwendungen:
- Verkleben von körpereigenem Hartgewebe, z.B. anatomische Reposition, Fixierung und Retention von Knochenfragmenten bei Trümmer- und Gelenkbrüchen
- Augmentation von Osteosynthesematerial aus Metall oder Kunststoff (Schrauben, Pins) im Knochen, besonders im osteoporotischen Knochen, zur Verbesserung der mechanischen Stabilität
- Einkleben von körpereigenem Weichgewebe in Hartgewebe, z.B. bei der Fixierung von Sehnen und Bändern in Knochenbohrlöchern
- Herstellung hochporöser Formkörper zur Implantation im Knochen
- Herstellung von Komposite-Werkstoffen durch Mischung mit keramischen oder salzartigen Stoffen wie z.B. Hydroxylapatit oder Calciumphosphaten
- Dentalmaterial, z.B. auch vorgefertigte Formkörper (Inserts)
- Zahnlack
- Grundstoff für implantierbare Wirkstoffträger
- resorbierbare Membran zur Abdeckung von Knochendefekten
- Material zur Erstellung von Implantaten zur Auffüllung größerer Knochendefekte z.B. nach Tumorresektion
- Material zur Verwendung von durch Rapid-Prototyping hergestellten resorbierbaren Implantaten

Es wurde nun gefunden, dass die Anforderungen der Technik, insbesondere die Anforderungen hinsichtlich Abbaubarkeit, Witterungsbeständigkeit, mechanischen und optischen Eigenschaften hervorragend erfüllt werden durch Polyester mit (Meth)acrylatendgruppen der allgemeinen Formel (I)

(I) CH₂=CRCOO-PE-OCOCR=CH₂

mit R = CH₃ oder H
mit PE = [-A-OCO-B-COO-]ₘ-A-, [-CHR'-COO-]ₙ-A-[-OCOCHR'-]ₙ,
worin
- A: für einen Alkyliden- mit 2-20 Kohlenstoffatomen bzw. Alkoxylidenrest mit 2-1.000 Alkoxylideneinheiten
- B: für einen gesättigten Alkylidenrest mit 2-10 Kohlenstoffatomen,
- R': für H oder CH₃, m für 0-100, n für 0-100 stehen,
mit der Maßgabe, dass die Summen m + n 2-200 beträgt, wobei die Polyester mit (Meth)acrylatendgruppen dadurch gekennzeichnet sind; dass
a) der Gehalt an kurzkettigen Di(meth)acrylaten (II)

   (II) CH₂=CRCOO-A-OCOCR=CH₂ < 5 Gew.% beträgt,
b) der Gehalt an Hydroxylgruppen < 0,25 Mol/Mol (Meth)acrylat liegt und
c) die Farbzahl nach APHA < 200 ist.

Dabei verhindert der niedrige Gehalt an kurzkettigen Di(meth)acrylaten (II) eine Versprödung der ansonsten weichen Netzwerke und gewährleistet die Abbaubarkeit zum Beispiel durch Hydrolyse der PE - Estergruppen.
Der geringe Gehalt an Hydroxylgruppen bedingt einerseits einen hohen Gehalt an vemetzungsaktiven Diestem, zum anderen bewirkt die Abwesenheit größerer Anteile Hydroxygruppen auch eine geringe Aufnahme von Wasser.
Schließlich garantiert die geringe Gelbfärbung die Synthese hochwertiger optischer Komponenten und ermöglicht z.B. im Bereich von Reparaturmassen eine gute und vor allem reproduzierbare Einfärbbarkeit.

Unter dem Begriff Polyester PE sind dabei Polyester und Oligoester in einem Molekulargewichtsbereich von 200-20.000, bevorzugt 300-8.000 und besonders bevorzugt im Bereich 400-4.000 Dalton zu verstehen. Hinsichtlich der Polyester sind solche auf Basis von α-Hydroxycarbonsäuren wie Glykolsäure und Milchsäure bevorzugt. Besonders bevorzugt sind Lactide. Andere Hydroxycarbonsäuren, wie z. B. Polyhydroxybuttersäure sind nicht erfindungsgemäß.
Im allgemeinen stehen m und n für Zahlen von 2-100, wobei insbesondere geradzahlige Werte für n bevorzugt sind. Besonderes Interesse gilt Oligolactiden im Bereich n= 2-20 und bevorzugt n=2-8. Für m sind Werte von 2-20, insbesondere 2-10 bevorzugt.

A ist der Alkylidenrest eines Alkandiols mit 2-20 Kohlenstoffatomen. Dieses gegebenenfalls verzweigte Alkandiol HO-A-OH enthält keine Doppelbindungen und abgesehen von maximal 3 Ethergruppen keine Heteroatome, insbesondere keine Stickstoffatome.

Als Beispiel für mögliche Alkandiole HO-A-OH sind zu nennen: Ethandiol, Di-, Tri- und Tetraehtylenglykol, Oligo- und Polyethylenglykole bis zu einem Molekulargewicht von bis zu 30.000 Dalton, Propylenglykol, 1,3-bzw. 1,4-Butandiol, 1,5-Pentandiol, 1,6-Hexandiol, 2,2-Dimehtylpropandiol-1,3 sowie 2,2,4-Trimethylhexandiol-1,6 und 2-Methylpropandiol- 1,3. Dabei sind Alkandiole ohne tert. CH-Gruppen bevorzugt.

B ist der Alkylidenrest einer gesättigten Dicarbonsäure. B steht für einen Alkylidenrest mit 2-10 Kohlenstoffatomen. Als Vertreter dieser Gruppe HOOC-B-COOH sind insbesondere zu nennen: Bersteinsäure, Glutarsäure, Adipinsäure, Azelainsäure, Sebacinsäure und Decandicarbonsäure, die gegebenenfalls durch Methylgruppen substituiert sind, bzw. Mischungen dieser Säuren.
Während im Falle der Polylactide Ethandiol oder Diethylenglykol besonders bevorzugt sind, ist im Falle der Polyester PE aus Dicarbonsäuren HOOC-B-COOH und Alkandiolen HO-A-OH Ethandiol weniger von Interesse. Hier sind insbesondere Butandiol, Hexandiol, Diethylenglykol und 2,2-Dimethylpropandiol-1,3 zu nennen. Besonders bevorzugt sind Polyester PE mit wenigstens 2 verschiedenen Alkandiolen HO-A-OH, wobei in der Regel eines der Alkandiole verzweigt ist. Als Beispiel ist zu nennen:
Poly(1,4-butandiol/2,2-Diemethylpropandiol-1,3) alt. adipinsäure.
In diesen Polyestern mit zwei verschiedenen Diolen verhindert der verzweigte Rest im allgemeinen die Kristallisation und gestattet so den Aufbau amorpher, hochtransparenter Produkte.
Prinzipiell können die Polyester aus Dicarbonsäuren HOOC-B-COOH und Alkandiolen HO-A-OH auch durch Hydroxycarbonsäuren, z. B. Milchsäure, modifiziert sein. Bevorzugt sind jedoch solche Polyester, die nur aus Dicarbonsäuren und Diolen aufgebaut sind.
Vom Prinzip her kann die Funktionalität der Polyester PE durch einen gewissen Anteil trifunktioneller Alkanole, z. B. Trimethylolpropan, erhöht werden. Im allgemeinen ist der Anteil derartiger trifunktioneller Verbindungen jedoch kleiner 10 %, bevorzugt < 1 %. Besonders bevorzugt sind Polyester PE, die keine trifunktionellen Alkanole enthalten.
Ein besonderes Charakteristikum der erfindungsgemäßen Polyester mit (Meth)acrylatendgruppen ist in den guten optischen Eigenschaften zu sehen.
So liegt der Gelbwert bzw. die Farbzahl APHA < 200, im allgemeinen < 100 bevorzugt < 50 (vergl. DIN 53409). Unter Umständen trägt der Verzicht auf jede Form von Heteroatomen, das Fehlen jedweder aromatischer Strukturen und leicht oxydierbarer Gruppen zu der besonderen Brillanz der erfindungsgemäßen Polyester mit Methacrylatendgruppen bei.
Wesentlich ist auch die Anzahl geeigneter Stabilisatoren, so sind insbesondere Hydrochinon oder Hydrochinonmonomethylether auf einen Gehalt < 100 ppm zu begrenzen. Besonders bevorzugt sind Polyester mit Methacrylatendgruppen, die <1 ppm dieser Phenole oder besser diese Phenole überhaupt nicht enthalten.

Die erfindungsgemäßen Polyester mit (Meth)acrylatendgruppen zeichnen sich auch durch einen besonders niedrigen Gehalt an Hydroxylgruppen aus. In erster Linie drückt dies den hohen Gehalt an (Meth)acrylolylendgruppen aus. Wesentlich für diese Polyester mit (Meth)acrylatendgruppen ist ein Verhältnis von freier Hydroxygruppe zu (Meth)acrylatendgruppe < 0,25 oder besser < 0,2 bzw. besonders bevorzugt < 0,15. Besonders gute Produkteigenschaften ergeben sich, wenn das Verhältnis freier Hydroxylgruppen zu Methacrylatendgruppen < 0,1 oder noch günstiger < 0,06 liegt. Im allgemeinen wird der Gehalt in freien Hydroxylgruppen durch die Hydroxylzahl bestimmt. Daneben sind auch andere Methoden, wie z. B. NMR geeignet. So wird z. B. die Bestimmung der Hydroxylzahl nach DIN ISO 4629 durchgeführt.
Im allgemeinen ist die Hydroxylzahl < 50mg KOH/g Polyester mit (Meth)acrylatendgruppen. Naturgemäß ist die Hydroxylzahl um so kleiner, je größer das Molekulargewicht des Polyesters mit (Meth)acrylatendgruppen ist. Als Daumenregel gilt folgendes:

| Molekulargewicht des Polyesters | Hydroxylzahl |
|---|---|
| (Mw) | (mg KOH/g Polyester (I)) |
| 500 | < 68 bevorzugt < 34 |
| 1000 | < 34 bevorzugt < 17 |
| 2000 | < 17 bevorzugt < 8 |
| 4000 | < 8 bevorzugt < 4 |

Von Bedeutung ist auch ein möglichst geringer Gehalt an kurzkettigen Diester (II). So verhindert ein möglichst geringer Gehalt dieser Diester (II) einerseits ein Verspröden der damit hergestellten Produkte, andererseits gewährleistet ein besonders niedriger Gehalt an (II) auch einen biologischen Abbau der Produkte. Darüber hinaus sind Polyester ohne niedrig siedende Komponenten sicherer in der Handhabung, vor allem zeigen sie keinerlei Geruchsbelästigung. Aus diesem Grunde ist auch der Gehalt an Lösungsmittel niedrig zu halten. Im allgemeinen ist der Gehalt an Lösungsmitteln < 20 Gew.%, bevorzugt < 5 Gew.%. Besonders bevorzugt enthält der erfindungsgemäße Polyester mit (Meth)acrylatendgruppen keinerlei Lösungsmittel.
Demgegenüber sind Polyester mit (Meth)acrylatendgruppen, die geringe Anteile an polymerisierbaren Monomeren enthalten, erfindungsgemäß. Hier ist als Monomeres insbesondere (Meth)acrylsäure zu nennen. Durch einen Gehalt von z. B. 0,5-10 Gew.% lässt sich einerseits die Rheologie der Polyester modifizieren, andererseits fördert der Gehalt an (Meth)acrylsäure bei der späteren Polymerisation die Cohäsion und die Adhäsion zu den verschiedensten Substraten.

Die bevorzugte Synthese der erfindungsgemäßen Polyester mit (Meth)acrylatendgruppen wird ohne Lösungsmittel durchgeführt. So wird in einem bevorzugten Verfahren ein Polyester HO-PE-OH in Gegenwart von 0,1-5 Gew.% eines sauren Katalysators mit Methacrylsäureanhydrid unter Bildung des Polyesters mit Methacrylatendgruppen (I) und Methacrylsäure umgesetzt.

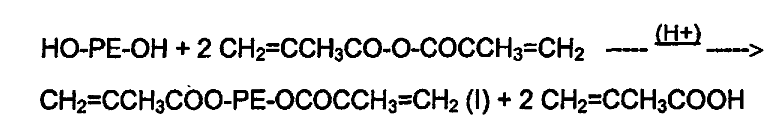

Wesentlich für diese Synthese ist die Anwesenheit der Katalysatorsäure in den oben genannten Konzentrationen, bevorzugt im Bereich 0,5-3 Gew.% und besonders bevorzugt im Bereich 0,8-2 Gew.% (jeweils bezogen auf das eingesetzte Methacrylsäureanhydrid).
Die Umsetzung erfolgt in einem Temperaturbereich von 0-100° C, bevorzugt im Bereich 20-80° C. In der Regel erfolgt die Reaktion innerhalb von 1-48 h und besonders bevorzugt im Bereich 4-24 h. Unter diesen Bedingungen werden die Estergruppen des Polyesters HO-PE-OH nicht gespalten, so dass auch keine freien Diole HO-A-OH entstehen, die schließlich nach Veresterung das unerwünschte Nebenprodukt (II) ergeben.
Wesentlich für eine möglichst vollständige Umsetzung ist dabei eine ausreichende Menge an Katalysatorsäure und ausreichend Zeit. Eine Temperaturerhöhung über 100° C hinaus fördert vor allem die Bildung von Nebenprodukten und ist daher zu vermeiden.
Als saure Katalysatoren verwendet man vorzugsweise Schwefelsäure, aromatische oder aliphatische Sulfonsäuren, die z. B. an ein Polymerharz gebunden sein können, oder Phosphonsäuren.

Als Ausgangsmaterial für die Synthese der erfindungsgemäßen Polyester mit (Meth)acrylatendgruppen ist insbesondere ein (Meth)-acrylsäureanhydrid geeignet, wie es durch Umsetzung von (Meth)acrylsäure mit Acetanhydrid erhältlich ist. Ein derartiges Methacrylsäureanhydrid ist beispielsweise in DE-OS 3510035 beschrieben. Üblicherweise enthält ein auf diesem Wege hergestelltes (Meth)acrylsäureanhydrid geringe Anteile an nicht umgesetzten Acetanhydrid sowie das gemischte Anhydrid aus (Meth)acrylsäure und Essigsäure.
Im allgemeinen ist eine Reinheit des (Meth)acrylsäureanhydrids von 93 % oder besser 96 % ausreichend. Bedingt durch den Gehalt an gemischtem Anhydrid und den Gehalt an Acetanhydrid erhält man bei der Umsetzung von HO-PE-OH mit dem (Meth)acrylsäureanhydrid geringe Anteile an Polyestem mit Essigsäureesterendgruppen. Es hat sich jedoch gezeigt, dass ein Gehalt an Polyestem mit Essigsäureestergruppen < 5 Gew.%, bevorzugt < 2 Gew.% und ganz besonders bevorzugt < 1 Gew.% tolerierbar ist. Im Unterschied zu chlorhaltigen Nebenprodukten, die in den erfindungsgemäßen Polyestem in der Regel auf einen Gehalt < 0,1 Gew.% zu beschränken sind oder besser gänzlich auszuschließen sind, sind Polyester, die als Nebenprodukt 0,2-5 Gew.% eines Polyesters mit Acetylendgruppen enthalten, erfindungsgemäß.

Im allgemeinen wird das molare Verhältnis von (Meth)acrylsäureanhydrid zu Hydroxylgruppe der Polyesterdiole HO-PE-OH im Bereich 1:1 gewählt. Bevorzugt ist jedoch der Einsatz eines geringen Unterschusses an (Meth)acrylsäureanhydrid (z. B. 0,95 Mol (Meth)acrylsäureanhydrid/1,0 Mol Hydroxylgruppe). Dies gewährleistet eine vollständige Umsetzung des (Meth)acrylsäureanhydrids, so dass eine Abtrennung von nicht umgesetztem Anhydrid entfällt.
Insbesondere bei höhermolekularen Polyesterdiolen ist es jedoch auch möglich, einen Überschuss an (Meth)acrylsäurehydrid, von z. B. 20 Mol %, zu verwenden. In diesem Fall wird nach möglichst vollständiger Umsetzung der Hydroxylgruppen des Polyesterdiols das überschüssige (Meth)acrylsäureanhydrid durch Zusatz eines niedermolekularen Alkohols wie Methanol, Ethanol oder Isopropanol zerstört.
Prinzipiell kann die bei der Umsetzung des Polyesterdiols mit dem (Meth)acrylsäurehydrid gebildete (Meth)acrylsäure direkt aus dem Reaktionsansatz destilliert werden. Daneben ist es auch möglich, die Umsetzung erst vollständig durchzuführen, den Katalysator abzutrennen und dann erst die (Meth)acrylsäure aus dem Ansatz zu destillieren. Diese Vorgehensweise bietet sich insbesondere bei polymergebundenen Katalysatorsäuren an. Auf jeden Fall erfolgt die Abtrennung der (Meth)acrylsäure bei reduziertem Druck, z. B. bei p < 10 mbar. Dabei wird zur besseren Stabilisierung Luft durch den Reaktionsansatz geleitet. Im allgemeinen wird die (Meth)acrylsäure bis auf einen Gehalt < 10 Gew.%, bevorzugt < 5 Gew.% und besonders bevorzugt < 2 Gew.% aus dem Reaktionsansatz entfernt.
Im Falle nicht polymergebundener Katalysatoren wie z. B. Schwefelsäure oder Methansulfonsäure kann das Entfernen des Katalysators auch durch Waschen mit Wasser entfernt werden. Die Abtrennung der (Meth)acrylsäure erfolgt durch Destillation.
Zur Verhinderung einer vorzeitigen Polymerisation werden bei der Umsetzung Polymerisationsinhibitoren zugesetzt, hierbei sind insbesondere sterisch gehinderte Phenole wie Topanol A oder lonol zu nennen. Hydrochinon oder Hydrochinonmonomethylether sind als Stabilisatoren ungeeignet.

Hinsichtlich der Polyesterdiole als Ausgangsmaterial kommen insbesondere die im Bereich der Polyurethanchemie verwendeten Polyesterdiole zum Einsatz mit den oben genannten Einschränkungen hinsichtlich A und B (siehe z. B. Ullmann's Encyclopedia of Industrial Chemistry, Vol. A 21, 228 bzw. 670). Dabei ist jedoch auf einen möglichst geringen Gehalt an freiem Alkandiol HO-A-OH zu achten. So soll der Gehalt an HO-A-OH im allgemeinen < 3 Gew.%, bevorzugt < 1 Gew.% und ganz besonders bevorzugt < 0,2 Gew.% liegen.

In der Regel sind Polyester mit (Meth)acrylendgruppen mit einer möglichst engen Molekulargewichtsverteilung besonders geeignet. Im allgemeinen sollte M_{w}/Mₙ < 2,5, bevorzugt < 1,8 und besonders bevorzugt < 1,5 liegen.

Erfindungsgemäße Umsetzungen mit Acrylsäureanhydrid sind aufgrund der außerordentlichen Polymerisationsreaktivität des Acrylats nur in bestenfalls sehr geringen Mengen in Mikroreaktoren durchzuführen.

In den folgenden Beispielen sind Ausführungsformen der Polyester näher erläutert:
Ganz allgemein lassen sich die erfindungsgemäßen Polyester mit (Meth)acrylatendgruppen dadurch erhalten, dass man die auf übliche Weise hergestellten Polyester sehr sorgfältig bei reduziertem Druck entgast, mit Nichtlösungsmittel extrahiert oder aus Lösungs/Fällungsmitteln umfällt, um so den Anteil am Di(meth)acrylat (II) auf < 5 Gew. % zu reduzieren.

### Beispiele

### Beispiel 1

### Synthese von Ethylenglykol-oligomilchsäure-dimethacrylat

PE = [-CHR-COO-]ₙ-A-[-OCOCHR-]ₙ

A = -CH₂-CH₂-, R = CH₃, n = 2

In einem 1 L Dreihalskolben werden 355 g Methacrylsäureanhydrid (stabilisiert mit 380 mg lonol) und 466 g Ethylenglykol-oligomilchsäure (M_{w} = 488 g/Mol) bei Raumtemperatur gemischt. Nach Zugabe von 6,5 g Methansulfonsäure wird unter Rühren langsam auf 60 °C erwärmt. Danach wird unter Erhöhung der Temperatur auf ca. 80°C die gebildete Methacrylsäure bei reduziertem Druck abdestilliert.
Das erhaltene farblose Öl wird mit Wasser, verdünnter Sodalösung und danach wieder mit Wasser gewaschen.

Zur Trocknung wird das im Produkt enthaltene Wasser im Vakuum abdestilliert.
Ausbeute 591 g farbloses, klares Öl
Farbzahl < 50 APHA
Ethylenglykoldimethacrylat: 0,17 %
Methacrylsäure: 0,87 %
Gehalt an Methacrylatendgruppen pro Ethylenglykol: 1,90
M_{W}: 605, M_{N}: 563, M_{W}/M_{N}: 1,07

### Vergleichsbeispiel 1 (nicht erfindungsgemäß)

Die gemäss Beispiel 1 eingesetzte Ethylenglykol-oligomilchsäure wird unter Verwendung von Methansulfonsäure als Katalysator mit Methacrylsäure unter Abziehen des entstehenden Wassers im Vakuum verestert.
Es resultiert ein braunes, trübes Öl
APHA-Farbzahl nicht anwendbar,
Ethylenglykoldimethacrylat: 5,6 %
Methacrylsäure: 4,3 %
Gehalt an Methacrylatendgruppen pro Ethylenglykol: 1,7

### Beispiel 2

Synthese von Poly-(1,4-butandiol/2,2-dimethylpropandiol-1,3-alt adipinsäure) mit Methacrylendgruppen.

PE = [-A-OCO-B-COO-]ₘ-A-

A = -CH₂-CH₂-CH₂-CH₂-, -CH₂-C(CH₃)₂-CH₂-

B = -CH₂-CH₂-CH₂-CH₂-

79,7 g Poly-(1,4-butandiol/2,2-dimethylpropandiol-1,3- alt. Adipinsäure) mit HO-Endgruppen (M_{N}: 750 g/Mol, Aldrich) wird mit 36,2 g Methacrylsäureanhydrid unter Zusatz von 0,7 g Methansulfonsäure als Katalysator umgesetzt. Nach Waschen mit Wasser und Abdestillieren der gebildeten Methacrylsäure resultiert ein glasklares, farbloses Öl.

### Beispiel 3

### Synthese von Diethylenglykol-oligomilchsäure-dimethacrylat

PE = [-CHR-COO-]ₙ-A-[-OCOCHR-]ₙ

A = -CH₂-CH₂-O-CH₂-CH₂-, R = CH₃, n = 2

In einem 1 L Dreihalskolben werden 330 g Methacrylsäureanhydrid (stabilisiert mit 320 mg lonol) und 441 g Diethylenglykol-oligomilchsäure (M_{w} = 479 g/Mol) bei Raumtemperatur gemischt. Nach Zugabe von 6,4 g Methansulfonsäure wird unter Rühren langsam auf 50 °C erwärmt. Danach wird unter Erhöhung der Temperatur auf ca. 70 °C die gebildete Methacrylsäure bei reduziertem Druck abdestilliert.
Das erhaltene farblose Öl wird mit Wasser, verdünnter Sodalösung und danach wieder mit Wasser gewaschen.
Zur Trocknung wird das im Produkt enthaltene Wasser im Vakuum abdestilliert.
Ausbeute 577 g farbloses, klares Öl
Diethylenglykoldimethacrylat: < 0,05
Methacrylsäure: 4,7 %
Gehalt an Methacrylatendgruppen pro Diethylenglykol: 1,45

### Beispiel 4:

### Synthese von Polyethylenglykol-600-oligomilchsäure-dimethacrylat

PE = [-CHR-COO-]ₙ-A-[-OCOCHR-]ₙ

A = -[CH₂-CH₂-O]ₙ-CH₂-CH₂- PEG-600

R = CH₃, n = 4

Man verfährt analog zu den Beipielen 1 und 3, verwendet jedoch folgende Einwaagen:
120,5 g Methacrylsäureanhydrid,
487,9 g Polyethylenglykol-600-oligomilchsäure (M_{w} = 1188 g/Mol)
250 mg lonol
4,3 g Methacrylsäureanhydrid
4,3 g Methansulfonsäure
Unter Rühren wird langsam auf 50 °C erwärmt. Danach werden unter Temperaturerhöhung auf 70 °C die flüchtigen Nebenprodukte unter reduziertem Druck abdestilliert.
Man erhält eine klare, farblose Flüssigkeit, die mit Wasser gewaschen und mit 63 mg lonol versetzt wird. Nach Trocknen im Vakuum erhält man 346 g farbloses, klares Öl.
Methacrylsäure: 4,7 %
Gehalt an Methacrylatendgruppen pro PEG-600 Einheit: 1,96
M_{W}:1221, M_{N}: 848, M_{W}/M_{N}: 1,44

## Patentansprüche

1. Polyester mit (Meth)acrylatendgruppen der allgemeinen Formel (I)
(I) CH₂=CRCOO-PE-OCOCR=CH₂
mit R = CH₃ bzw. H
mit PE = [-A-OCO-B-COO-]ₘ-A-, [-CHR'-COO-]ₙ-A-[-OCOCHR'-]ₙ,
worin
A für einen Alkyliden- mit 2-20 Kohlenstoffatomen bzw. Alkoxylidenrest mit 2-1.000 Alkoxylideneinheiten
B für einen gesättigten Alkylidenrest mit 2-10 Kohlenstoffatomen,
R' für H oder CH₃,
m für 0-100, n für 0-100 stehen,
mit der Maßgabe, dass die Summen m + n 2-200 beträgt, **dadurch gekennzeichnet, dass**
a) der Gehalt an kurzkettigen Di(meth)acrylaten
(II) CH₂=CCH₃COO-A-OCOCCH₃=CH₂ < 5 Gew.% beträgt,
b) der Gehalt an Hydroxylgruppen < 0,25 Mol/Mol (Meth)acrylat ist,
c) die Farbzahl nach APHA < 200 liegt.

2. Polyester mit (Meth)acrylatendgruppen gemäss Anspruch 1, **dadurch gekennzeichnet, dass** PE für einen Polyester vom Typ [-A-OCO-B-COO]ₘ-A- steht.

3. Polyester mit (Meth)acrylatendgruppen gemäss Anspruch 1, **dadurch gekennzeichnet, dass** PE für einen Polyester vom Typ [-CHR-COO]ₙ-A-[-OCOCHR-]ₙ steht.

4. Polyester mit (Meth)acrylatendgruppen gemäss einem der Ansprüche 1 bis 3, **dadurch gekennzeichnet, dass** der Polyester 0,5 - 10 Gew.% (Meth)acrylsäure enthält.

5. Polyester mit (Meth)acrylatendgruppen gemäss einem der Ansprüche 1 bis 4, **dadurch gekennzeichnet, dass** der Polyester ein Molekulargewicht zwischen 400 und 4000 Dalton besitzt.

6. Polyester mit (Meth)acrylatendgruppen gemäss einem der Ansprüche 1 bis 5, **dadurch gekennzeichnet, dass** der Polyester ein Lactid ist.

7. Polyester mit (Meth)acrylatendgruppen gemäss einem der Ansprüche 1 bis 6, **dadurch gekennzeichnet, dass** der Polyester keine trifunktionellen Alkohole enthält.

8. Polyester mit (Meth)acrylatendgruppen gemäss einem der Ansprüche 1 bis 7, **dadurch gekennzeichnet, dass** der Gehalt an Hydroxylgruppen kleiner 0.20 Mol/Mol (Meth)acrylat ist

9. Polyester mit (Meth)acrylatendgruppen gemäss einem der Ansprüche 1 bis 8, **dadurch gekennzeichnet, dass** die Farbzahl APHA kleiner 50 beträgt.

10. Polyester mit (Meth)acrylatendgruppen gemäss einem der Ansprüche 1 bis 9, **dadurch gekennzeichnet, dass** die Molekulargewichtsverteilung M_{w}/M_{N} < 2.5 ist.

11. Verfahren zur Herstellung der Polyester mit (Meth)acrylatendgruppen gemäss einem der Ansprüche 1-10, **dadurch gekennzeichnet, dass** ein Diol (III) HO-PE-OH
mit (Meth)acrylsäureanhydrid unter Bildung von (I) und (Meth)acrylsäure umgesetzt wird.

12. Verfahren zur Herstellung der Polyester mit (Meth)acrylatendgruppen gemäss einem der Ansprüche 1-10, **dadurch gekennzeichnet, dass** die Synthese in Anwesenheit eines sauren Katalysators mit einer Konzentration von 0.1-5 Gew.% durchgeführt wird.

13. Verwendung von Polyestern gemäss einem der Ansprüche 1-10 zur Herstellung von Medizinprodukten für die Endoprothetik, Osteosynthese und Wundheilung.

14. Verwendung gemäss Anspruch 13, **dadurch gekennzeichnet, dass** die Polyester zum Verkleben von körpereigenem Hart- und/oder Weichgewebe eingesetzt werden.

15. Verwendung gemäss Anspruch 13, **dadurch gekennzeichnet, dass** die Polyester zur Augmentation von Osteosynthesematerial aus Metall oder Kunststoff im Knochen dienen.

16. Verwendung gemäss Anspruch 13, **dadurch gekennzeichnet , dass** die Polyester zur Herstellung hochporöser Formkörper zur Implantation im Knochen dienen.

17. Verwendung gemäss Anspruch 13, **dadurch gekennzeichnet, dass** die Polyester zur Herstellung von Komposite-Werkstoffen eingesetzt werden.

18. Verwendung gemäss Anspruch 13, **dadurch gekennzeichnet , dass** die Polyester als Dentalmaterial wie Zahnlack oder Inserts eingesetzt werden.

19. Verwendung gemäss Anspruch 13, **dadurch gekennzeichnet , dass** die Polyester als resorbierbare Membran zur Abdeckung von Knochendefekten eingesetzt werden.

## Claims

1. Polyester containing (meth)acrylate end groups of the general formula (I)
(I) CH₂=CRCOO-PE-OCOCR=CH₂
where R = CH₃ or H,
where PE = [-A-OCO-B-COO-]ₘ-A-, [-CHR'-COO-]ₙ-A-[-OCOCHR'-]ₙ,
in which
A is an alkylidene radical having 2-20 carbon atoms or an alkoxylidene radical having 2-1000 alkoxylidene units,
B is a saturated alkylidene radical having 2-10 carbon atoms,
R' is H or CH₃,
m is 0-100, n is 0-100,
with the proviso that the sum m + n is 2-200, **characterised in that**
a) the content of short-chain di(meth)acrylates
(II) CH₂=CCH₃COO-A-OCOCCH₃=CH₂ is < 5% by weight,
b) the content of hydroxyl groups is < 0.25 mol/mol of (meth)acrylate, and
c) the APHA colour number is < 200.

2. Polyester containing (meth)acrylate end groups according to Claim 1, **characterised in that** PE is a polyester of the [-A-OCO-B-COO]ₘ-A- type.

3. Polyester containing (meth)acrylate end groups according to Claim 1, **characterised in that** PE is a polyester of the [-CHR-COO]ₙ-A-[-OCOCHR-]ₙ type.

4. Polyester containing (meth)acrylate end groups according to one of Claims 1 to 3, **characterised in that** the polyester comprises 0.5-10% by weight of (meth)acrylic acid.

5. Polyester containing (meth)acrylate end groups according to one of Claims 1 to 4, **characterised in that** the polyester has a molecular weight of between 400 and 4000 daltons.

6. Polyester containing (meth)acrylate end groups according to one of Claims 1 to 5, **characterised in that** the polyester is a lactide.

7. Polyester containing (meth)acrylate end groups according to one of Claims 1 to 6, **characterised in that** the polyester comprises no trifunctional alcohols.

8. Polyester containing (meth)acrylate end groups according to one of Claims 1 to 7, **characterised in that** the content of hydroxyl groups is less than 0.20 mol/mol of (meth)acrylate.

9. Polyester containing (meth)acrylate end groups according to one of Claims 1 to 8, **characterised in that** the APHA colour number is less than 50.

10. Polyester containing meth)acrylate end groups according to one of Claims 1 to 9, **characterised in that** the molecular weight distribution M_{w}/Mₙ is < 2.5.

11. Process for the preparation of polyesters containing (meth)acrylate end groups according to one of Claims 1-10, **characterised in that** a diol
(III) HO-PE-OH
is reacted with (meth)acrylic anhydride with formation of (I) and (meth)-acrylic acid.

12. Process for the preparation of polyesters containing (meth)acrylate end groups according to one of Claims 1-10, **characterised in that** the synthesis is carried out in the presence of an acidic catalyst with a concentration of 0.1-5% by weight.

13. Use of polyesters according to one of Claims 1-10 for the production of medical products for endoprosthetics, osteosynthesis and wound healing.

14. Use according to Claim 13, **characterised in that** the polyesters are employed for the adhesive bonding of endogenous hard and/or soft tissue.

15. Use according to Claim 13, **characterised in that** the polyesters serve for augmentation of metal or plastic osteosynthesis material in bone.

16. Use according to Claim 13, **characterised in that** the polyesters serve for the production of highly porous mouldings for implantation in bone.

17. Use according to Claim 13, **characterised in that** the polyesters are employed for the production of composite materials.

18. Use according to Claim 13, **characterised in that** the polyesters are employed as dental material, such as dental varnish or inlays.

19. Use according to Claim 13, **characterised in that** the polyesters are employed as absorbable membrane for covering bone defects.

## Revendications

1. Polyester contenant des groupements (méth)acrylate terminaux de formule générale (I)
(I) CH₂=CRCOO-PE-OCOCR=CH₂
où R = CH₃ ou H,
où PE = [-A-OCO-B-COO-]ₘ-A-, [-CHR'-COO-]ₙ-A-[-OCOCHR'-]ₙ,
dans laquelle
A représente un radical alkylidène ayant de 2 à 20 atomes de carbone ou un radical alkoxylidène ayant de 2 à 1000 motifs alkoxylidène,
B représente un radical alkylidène saturé ayant de 2 à 10 atomes de carbone,
R' représente H ou CH₃,
m vaut 0-100, n vaut 0-100,
à condition que la somme m + n vaille 2-200, **caractérisé en ce que**
a) la teneur en di(méth)acrylates de chaîne courte
(II) CH₂=CCH₃COO-A-OCOCCH₃=CH₂ est < 5% en poids,
b) la teneur en groupements hydroxyle est < 0,25 mol/mol de (méth)acrylate, et
c) l'indice de couleur APHA est < 200.

2. Polyester contenant des groupements (méth)acrylate terminaux selon la revendication 1, **caractérisé en ce que** PE est un polyester de type [-A-OCO-B-COO]ₘ-A-.

3. Polyester contenant des groupements (méth)acrylate terminaux selon la revendication 1, **caractérisé en ce que** PE est un polyester de type [-CHR-COO]ₙ-A-[-OCOCHR-]ₙ.

4. Polyester contenant des groupements (méth)acrylate terminaux selon l'une des revendications 1 à 3, **caractérisé en ce que** le polyester comprend de 0,5 à 10% en poids d'acide (méth)acrylique.

5. Polyester contenant des groupements (méth)acrylate terminaux selon l'une des revendications 1 à 4, **caractérisé en ce que** le polyester a un poids moléculaire compris entre 400 et 4000 daltons.

6. Polyester contenant des groupements (méth)acrylate terminaux selon l'une des revendications 1 à 5, **caractérisé en ce que** le polyester est un lactide.

7. Polyester contenant des groupements (méth)acrylate terminaux selon l'une des revendications 1 à 6, **caractérisé en ce que** le polyester ne comprend aucun alcool trifonctionnel.

8. Polyester contenant des groupements (méth)acrylate terminaux selon l'une des revendications 1 à 7, **caractérisé en ce que** la teneur en groupements hydroxyle est inférieure à 0,20 mol/mol de (méth)acrylate.

9. Polyester contenant des groupements (méth)acrylate terminaux selon l'une des revendications 1 à 8, **caractérisé en ce que** l'indice de couleur APHA est inférieur à 50.

10. Polyester contenant des groupements (méth)acrylate terminaux selon l'une des revendications 1 à 9, **caractérisé en ce que** la distribution de poids moléculaire M_{w}/Mₙ est < 2,5.

11. Procédé de préparation de polyesters contenant des groupements (méth)acrylate terminaux selon l'une des revendications 1 à 10, **caractérisé en ce qu'**un diol
(III) HO-PE-OH
est réagi avec un anhydride (méth)acrylique avec la formation de (I) et d'acide (méth)acrylique.

12. Procédé de préparation of polyesters contenant des groupements (méth)acrylate terminaux selon l'une des revendications 1 à 10, **caractérisé en ce que** la synthèse est réalisée en présence d'un catalyseur acide ayant une concentration de 0,1-5% en poids.

13. Utilisation de polyesters selon l'une des revendications 1 à 10 pour la production de produits médicaux pour les endoprothèses, l'ostéo-synthèse et la guérison de plaies.

14. Utilisation selon la revendication 13, **caractérisé en ce que** les polyesters sont employés pour la liaison adhésive de tissus endogènes durs et/ou mous.

15. Utilisation selon la revendication 13, **caractérisé en ce que** les polyesters servent à augmenter le matériau d'ostéosynthèse métallique ou plastique dans l'os.

16. Utilisation selon la revendication 13, **caractérisé en ce que** les polyesters servent à la production de moulages hautement poreux pour une implantation dans l'os.

17. Utilisation selon la revendication 13, **caractérisé en ce que** les polyesters sont employés pour la production de matériaux composites.

18. Utilisation selon la revendication 13, **caractérisé en ce que** les polyesters sont employés comme matériau dentaire, tel que les incrustations ou le vernis dentaires.

19. Utilisation selon la revendication 13, **caractérisé en ce que** les polyesters sont employés comme membrane absorbable pour recouvrir des défauts de l'os.
